# EUROPÄISCHE PATENTANMELDUNG

(11) **EP 0 654 465 A1**
(43) Veröffentlichungstag der Anmeldung: **24.05.1995**
(21) Anmeldenummer: 94810643.0
(22) Anmeldetag: 08.11.1994
(51) Int. Cl.: C07C 233/78, C07C 233/79, C07C 233/80, C08G 59/54, C07C 233/59, C07C 233/40, C07C 233/41, C07C 235/10, C08L 63/00

(54) **Polyaminopolyamide**

(30) Priorität: 15.11.1993 CH 3414/93
(71) Anmelder: CIBA-GEIGY AG, CH-4002 Basel (CH)
(72) Erfinder: Fischer, Walter, Dr., CH-4153 Reinach (CH); Helbling, Christine, CH-4127 Birsfelden (CH)

(57) **Zusammenfassung**

Polyaminopolyamide der Formel I 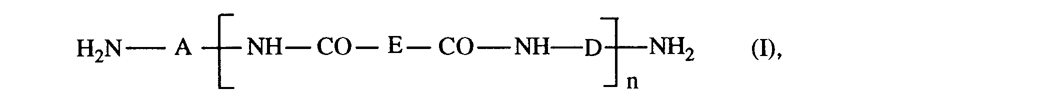
worin n eine Zahl von 1 bis 4 bedeutet,
A und D gleich oder verschieden sind und unabhängig voneinander je für einen Rest der Formeln 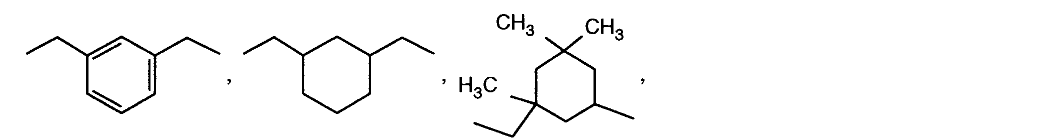 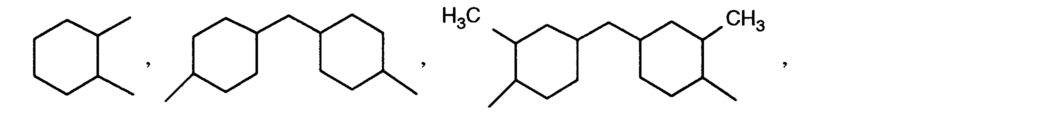 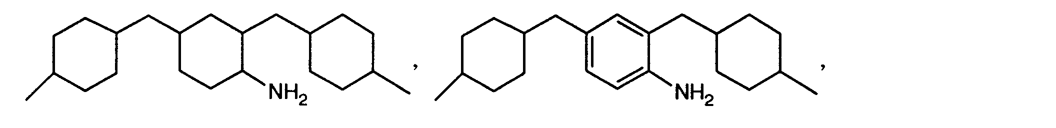 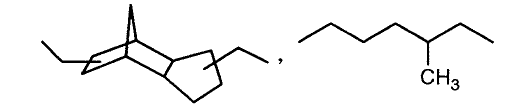
oder für ein Isomerengemisch aus den beiden Resten Formel 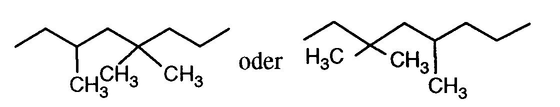
stehen und
E einen Rest der Formel 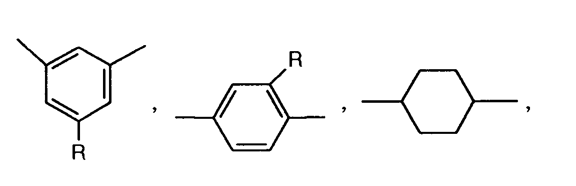 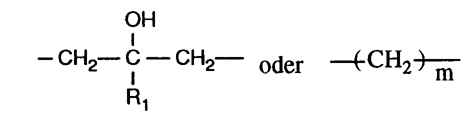
bedeutet, wobei R für ein Wasserstoffatom oder einen Rest der Formel -CO-NH-A-NH₂ oder -CO-NH-D-NH₂ steht, R₁ für einen Rest der Formel -CO-NH-A-NH₂ oder -CO-NH-D-NH₂ steht und m eine Zahl von 2 bis 12 bedeutet, eignen sich zum Härten von Epoxidharzen, vorzugsweise für die Kalthärtung.

## Beschreibung

Die vorliegende Erfindung betrifft neue Polyaminoamide, die durch Umsetzung bestimmter Di- oder Tricarbonsäuren oder deren Ester mit bestimmten Di- oder Triaminen erhalten werden, härtbare Epoxidharzgemische enthaltend die neuen Polyaminoamide und die aus den härtbaren Epoxidharzgemischen durch Härtung erhaltenen Formstoffe, inbesondere Beschichtungen.

Zur Herstellung von chemikalienresistenten Beschichtungen auf Basis von Epoxidharzen werden als Härtungsmittel vor allem flüssige Polyamine eingesetzt, wie beispielsweise formulierte Gemische auf Basis von 4,4'-Diaminodiphenylmethan. Dieser Härter gilt aber als mutagen- und kanzerogenverdächtig und wird beispielsweise in USA seit Dezember 1992 mit aussergewöhnlichen Anwendungsauflagen seitens der staatlichen Behörden OSHA (Occupational Safety and Health Administration), Final Rule in the Federal Register on the Use of Methylene Dianiline, belegt. In ähnlicher Weise hat das schweizerische Bundesamt für Gesundheitswesen diesen Härter gemäss Giftliste 1 (Ausgabe 1991) in die Giftklasse 1* (kanzerogen) eingestuft. Es besteht somit ein Bedarf nach anderen aminischen Härtungsmitteln für Epoxidharzbeschichtungen.

Ein von der Firma Anchor Chemical unter der Bezeichnung Ancamine X2280 im Handel erhältliches flüssiges Polyamin auf Basis von polycyclischen Polyaminen eignet sich zur Herstellung von Epoxidharzbeschichtungen mit einer guten Chemikalienresistenz, mit Ausnahme gegenüber von Carbonsäuren, wie beispielsweise Essigsäure.

In der EP-A-0 286 933 werden als Härtungsmittel für Epoxidharze unter anderem auch Umsetzungsprodukte aus Malonsäureestern mit Polyaminen vorgeschlagen. Solche Umsetzungsprodukte werden in den Beispielen der EP-A-0 286 933 weder eingesetzt noch werden deren Herstellung und Eigenschaften beschrieben.

Es wurde nun gefunden, dass bestimmte neue Polyaminoamide, die durch Umsetzung bestimmter Di- oder Tricarbonsäuren oder deren Ester mit bestimmten Di- oder Triaminen erhalten werden, den Epoxidharzbeschichtungen eine bessere Chemikalienresistenz, inbesondere gegenüber Carbonsäuren, verleihen.

Gegenstand vorliegender Erfindung sind somit Polyaminopolyamide der Formel I 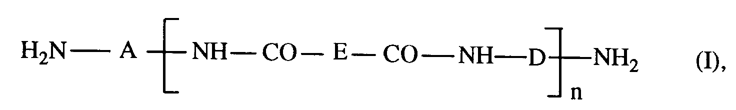
worin n eine Zahl von 1 bis 4 bedeutet,
A und D gleich oder verschieden sind und unabhängig voneinander je für einen Rest der Formeln 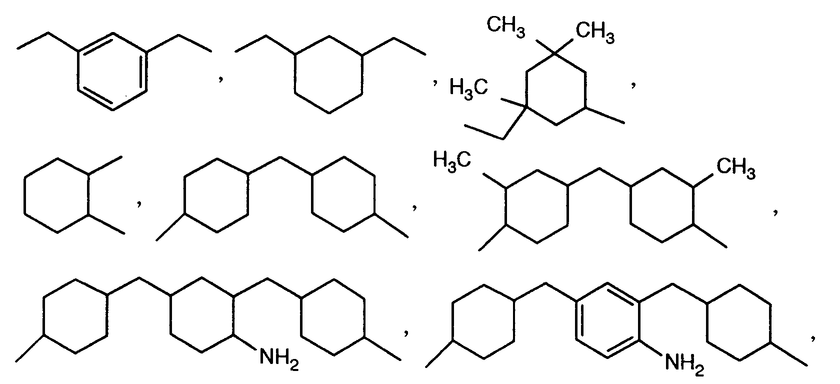 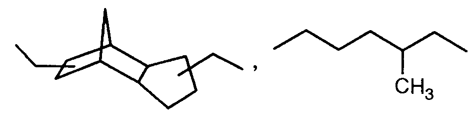
oder für ein Isomerengemisch aus den beiden Resten der Formel 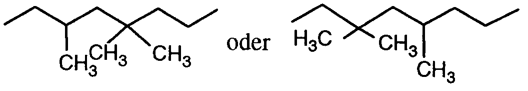
stehen und
E einen Rest der Formel 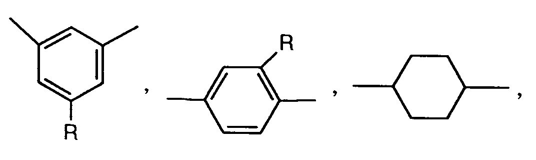 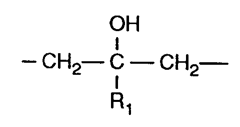
oder (̵CH₂ 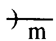 bedeutet, wobei R für ein Wasserstoffatom oder einen Rest der Formel -CO-NH-A-NH₂ oder -CO-NH-D-NH₂ steht, R₁ für einen Rest der Formel -CO-NH-A-NH₂ oder -CO-NH-D-NH₂ steht und m eine Zahl von 2 bis 12 bedeutet.

Vorzugsweise betrifft die Erfindung Polyaminopolyamide der Formel I, worin n eine Zahl von 1 bis 4 bedeutet,
A und D gleich oder verschieden sind und unabhängig voneinander je für einen Rest der Formeln 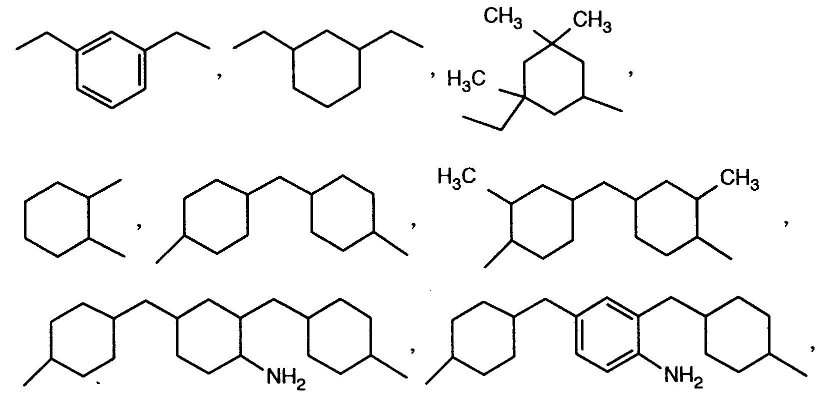 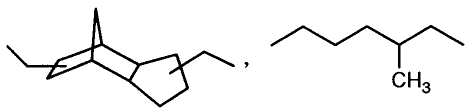
oder für ein Isomerengemisch aus den 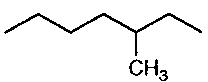
oder für ein Isomerengemisch aus den beiden Resten der Formel 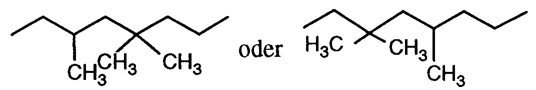
stehen und E einen Rest der Formel 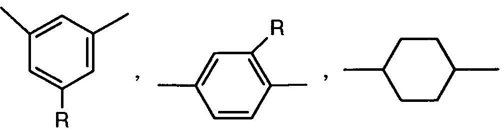
oder (̵CH₂ 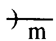 bedeutet, wobei R für ein Wasserstoffatom steht und m eine Zahl von 4 bis 12, vorzugsweise 6 oder 8, bedeutet.

Von den erfindungsgemässen Polyaminoamiden stellen solche der Formel I, worin E einen Rest der Formel 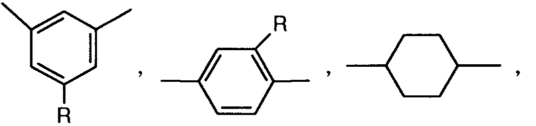 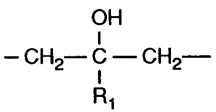
oder (̵CH₂ 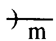 bedeutet, wobei R für ein Wasserstoffatom steht, R₁ für einen Rest der Formel -CO-NH-A-NH₂ oder -CO-NH-D-NH₂ steht und m eine Zahl von 4 bis 12, vorzugsweise die Zahl 4, 6 oder 8, bedeutet, eine weitere bevorzugte Gruppe von Verbindungen dar.

Insbesondere bedeutet dabei E einen Rest der Formeln 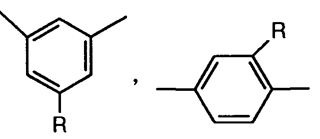
oder 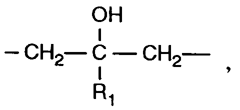
worin R für ein Wasserstoffatom steht und R₁ für einen Rest der Formel -CO-NH-A-NH₂ oder -CO-NH-D-NH₂ steht.

In den erfindungsgemässen Polyaminoamiden bedeuten A und D vorzugsweise unabhängig voneinander je einen Rest der Formel 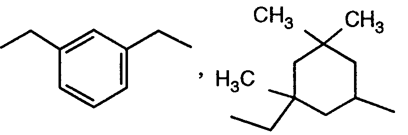 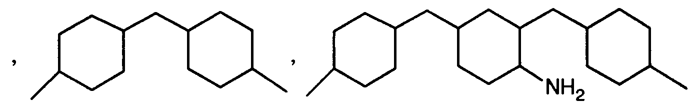
oder

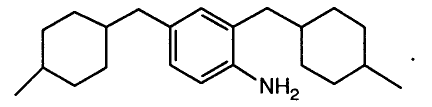

Insbesondere haben A und D in der Formel I die gleiche Bedeutung.

In der Formel I steht der Index n vorzugsweise für die Zahl 1.

Wie eingangs erwähnt, stellen die erfindungsgemässen Verbindungen vorteilhafte aminische Härtungsmittel für Epoxidharze dar.

Gegenstand vorliegender Erfindung ist somit auch ein Härtungsmittel für Epoxidharze, das dadurch gekennzeichnet ist, dass es mindestens eine Verbindung der Formel I enthält.

Das erfindungsgemässe Härtungsmittel kann beispielsweise hergestellt werden, indem man eine Di- oder Tricarbonsäure oder deren Mono-, Di- oder Triester der Formel II

R²OOC―E―COOR² (II),

worin E einen Rest der Formel 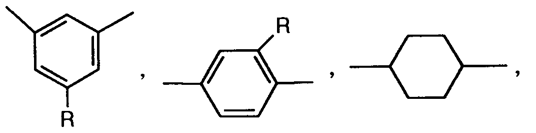 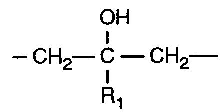
oder (̵CH₂ 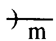 bedeutet, wobei R für ein Wasserstoffatom oder einen Rest der Formel -CO-NH-A-NH₂ oder -CO-NH-D-NH₂ steht, R₁ für einen Rest der Formel -CO-NH-A-NH₂ oder -CO-NH-D-NH₂ steht und m eine Zahl von 2 bis 12 bedeutet, mit einem Di- oder Triamin der Formel III und IV

H₂N―A―NH₂ (III) und H₂N―D―NH₂ (IV),

worin A und D die gleiche Bedeutung wie in Formel I haben, bei erhöhter Temperatur umsetzt, wobei man auf 1 Mol der Verbindung der Formel II 2 bis 20 Mole der Verbindungen der Formeln III und IV einsetzt.

Im allgemeinen wird diese Umsetzung im Temperaturbereich von 80° bis 250°C vorgenommen. Vorzugsweise wendet man bei der Umsetzung der Di- und Tricarbonsäuren mit den Di- oder Triaminen Temperaturen von 180 bis 250°C und bei der Umsetzung der Di- und Tricarbonsäureester mit den Di- oder Triaminen Temperaturen von 100 bis 180°C an.

Vorzugsweise liegen bei der Umsetzung die Di- oder Triamine der Formeln III und IV in einem äquivalenten Überschuss vor, so dass bei der Umsetzung im wesentlichen die entsprechenden Diaminodicarbonsäurediamide und Triaminotricarbonsäuretriamide neben einem Überschuss an Di- und Triamin entstehen. Für den Einsatz dieser Umsetzungsprodukte als Härtungsmittel ist es möglich, aber nicht erforderlich, die Umsetzungsprodukte von dem überschüssigen Di- oder Triamin zu befreien.

Die Umsetzung von Di- und Tricarbonsäuren bzw. deren Estern mit den Diaminen kann auch in Gegenwart von Zusätzen, wie Lösungsmitteln, beispielsweise Butanol oder Xylol, Beschleunigern, beispielsweise Salicylsäure, Nonylphenol, Alkali- oder Erdalkaliperchlorate oder -nitrate, Flexibilisatoren, beispielsweise Polyethylenglykole, Fliessmittel, beispielsweise Benzylalkohol, Pigmenten, Füllstoffen oder Antioxidantien, durchgeführt werden.

Die Umsetzung kann auch gleichzeitig oder nacheinander mit verschiedenen Diaminen und verschiedenen Di- und Tricarbonsäuren oder deren Estern durchgeführt werden, wobei die entsprechenden Produktegemische erhalten werden.

Wie eingangs erwähnt, können die erfindungsgemässen Polyaminoamide zum Härten von üblichen Epoxidharzen eingesetzt werden.

Ein weiterer Gegenstand vorliegender Erfindung sind somit auch härtbare Gemische, enthaltend
(a) ein Epoxidharz mit mehr als einer 1,2-Epoxidgruppe im Molekül und
(b) mindestens eine erfindungsgemässe Verbindung der Formel I.

Als Epoxidharze (a), die in den erfindungsgemässen härtbaren Gemischen eingesetzt werden können, eignen sich die in der Epoxidharztechnik üblichen Epoxidharze. Beispiele für Epoxidharze sind:
I) Polyglycidyl- und Poly-(β-methylglycidyl)-ester, erhältlich durch Umsetzung einer Verbindung mit mindestens zwei Carboxylgruppen im Molekül und Epichlorhydrin bzw. β-Methylepichlorhydrin. Die Umsetzung erfolgt zweckmässig in der Gegenwart von Basen.
   Als Verbindung mit mindestens zwei Carboxylgruppen im Molekül können aliphatische Polycarbonsäuren verwendet werden. Beispiele für solche Polycarbonsäuren sind Oxalsäure, Bernsteinsäure, Glutarsäure, Adipinsäure, Pimelinsäure, Sebacinsäure, Korksäure, Azelainsäure oder dimerisierte bzw. trimerisierte Linolsäure.
   Es können aber auch cycloaliphatische Polycarbonsäuren eingesetzt werden, wie beispielsweise Tetrahydrophthalsäure, 4-Methyltetrahydrophthalsäure, Hexahydrophthalsäure oder 4-Methylhexahydrophthalsäure.
   Weiterhin können aromatische Polycarbonsäuren Verwendung finden, wie beispielsweise Phthalsäure, Isophthalsäure oder Terephthalsäure.
II) Polyglycidyl-oder Poly-(β-methylglycidyl)-ether, erhältlich durch Umsetzung einer Verbindung mit mindestens zwei freien alkoholischen Hydroxygruppen und/oder phenolischen Hydroxygruppen und Epichlorhydrin oder β-Methylepichlorhydrin unter alkalischen Bedingungen, oder in Anwesenheit eines sauren Katalysators und anschliessende Alkalibehandlung.
   Die Glycidylether dieses Typs leiten sich beispielsweise von acyclischen Alkoholen ab, wie von Ethylenglykol, Diethylenglykol und höheren Poly-(oxyethylen)-glykolen, Propan-1,2-diol oder Poly-(oxypropylen)-glykolen, Propan-1,3-diol, Butan-1,4-diol, Poly-(oxytetramethylen)-glykolen, Pentan-1,5-diol, Hexan-1,6-diol, Hexan-2,4,6-triol, Glycerin, 1,1,1-Trimethylolpropan, Pentaerythrit, Sorbit, sowie von Polyepichlorhydrinen. Sie leiten sich aber auch beispielsweise von cycloaliphatischen Alkoholen, wie 1,4-Cyclohexandimethanol, Bis-(4-hydroxycyclohexyl)-methan oder 2,2-Bis-(4-hydroxycyclohexyl)-propan, ab oder sie besitzen aromatische Kerne, wie N,N-Bis-(2-hydroxyethyl)-anilin oder p,p'-Bis-(2-hydroxyethylamino)-diphenylmethan.
   Die Glycidylether können sich auch von einkerningen Phenolen ableiten, wie beispielsweise von Resorcin oder Hydrochinon, oder sie basieren auf mehrkernigen Phenolen, wie beispielsweise Bis-(4-hydroxyphenyl)-methan, 4,4'-Dihydroxybiphenyl, Bis-(4-hydroxyphenyl)-sulfon, 1,1,2,2-Tetrakis-(4-hydroxyphenyl)-ethan, 2,2-Bis-(4-hydroxyphenyl)-propan, 2,2-Bis-(3,5-dibrom-4-hydroxyphenyl)-propan sowie von Novolaken, erhältlich durch Kondensation von Aldehyden, wie Formaldehyd, Acetaldehyd, Chloral oder Furfuraldehyd, mit Phenolen, wie Phenol, oder mit Phenolen, die im Kern mit Chloratomen oder C₁-C₉-Alkylgruppen substituiert sind, wie beispielsweise 4-Chlorphenol, 2-Methylphenol, oder 4-tert.-Butylphenol oder durch Kondensation mit Bisphenolen, wie solche der oben genannten Art.
III) Poly-(N-glycidyl)-verbindungen, erhältlich durch Dehydrochlorierung der Reaktionsprodukte von Epichlorhydrin mit Aminen, die mindestens zwei Aminwasserstoffatome enthalten. Bei diesen Aminen handelt es sich zum Beispiel um Anilin, n-Butylamin, Bis-(4-aminophenyl)-methan, m-Xylylendiamin oder Bis-(4-methylaminophenyl)-methan. Zu den Poly-(N-glycidyl)-verbindungen zählen aber auch Triglycidylisocyanurat, N,N'-Diglycidylderivate von Cycloalkylenharnstoffen, wie Ethylenharnstoff oder 1,3-Propylenharnstoff, und Diglycidylderivate von Hydantoinen, wie von 5,5-Dimethylhydantoin.
IV) Poly-(S-glycidyl)-verbindungen, beispielsweise Di-S-glycidylderivate, die sich von Dithiolen, wie beispielsweise Ethan-1,2-dithiol oder Bis-(4-mercaptomethylphenyl)-ether ableiten.
V) Cycloaliphatische Epoxidharze, beispielsweise Bis-(2,3-epoxycyclopentyl)-ether, 2,3-Epoxycyclopentylglycidylether, 1,2-Bis-(2,3-epoxycyclopentyloxy)-ethan oder 3,4-Epoxycyclohexylmethyl-3',4'-epoxycyclohexancarboxylat.

Es lassen sich aber auch Epoxidharze verwenden, bei denen die 1,2-Epoxidgruppen an unterschiedliche Heteroatome bzw. funktionelle Gruppen gebunden sind; zu diesen Verbindungen zählen beispielsweise das N,N,O-Triglycidylderivat des 4-Aminophenols, der Glycidylether-glycidylester der Salicylsäure, N-Glycidyl-N'-(2-glycidyloxypropyl)-5,5-dimethylhydantoin oder 2-Glycidyloxy-1,3-bis-(5,5-dimethyl-1-glycidylhydantoin-3-yl)-propan.

Bevorzugt verwendet man in den erfindungsgemässen härtbaren Gemischen als Epoxidharz (a) einen flüssigen oder viskosen Polyglycidylether oder -ester, insbesondere einen flüssigen oder viskosen Bisphenoldiglycidylether.

Die oben genannten Epoxidverbindungen sind bekannt und zum Teil im Handel erhältlich. Es können auch Gemische von Epoxidharzen verwendet werden.

Die Menge des eingesetzten Härtungsmittels richtet sich nach der chemischen Natur des Härtungsmittels und nach den gewünschten Eigenschaften der härtbaren Mischung und des gehärteten Produktes. Die maximale Menge kann leicht ermittelt werden. Normalerweise werden 0,75 bis 1,25 Äquivalente Aminwasserstoff pro 1 Epoxidäquivalent eingesetzt.

Die Herstellung der erfindungsgemässen Gemische kann in üblicher Weise durch Vermischen der Komponenten durch Handrührung oder mit Hilfe bekannter Mischaggregate, beispielsweise mittels Rührer, Kneter oder Walzen, erfolgen.

Je nach Anwendung können den erfindungsgemässen Gemischen die gebräuchlichen Zusätze beigegeben werden, wie beispielsweise Füllstoffe, Pigmente, Farbstoffe, Verlaufmittel oder Plastifizierungsmittel.

Die erfindungsgemässen Gemische zeichnen sich vorteilhaft durch eine vergleichsweise geringere Carbonatisierungstendenz auf, dass heisst, in der Kälte und bei hoher Luftfeuchtigkeit tritt in den Gemischen keine Trübung durch Aufnahme von CO₂ aus der Luft und Kristallbildung nach Umsetzung des CO₂ mit den Polyaminen auf.

Die Härtung der erfindungsgemässen Gemische kann in an sich bekannter Weise ein- oder mehrstufig vorgenommen werden. Die Härtung erfolgt im allgemeinen bei Raumtemperatur oder unterhalb der Raumtemperatur oder durch Erhitzen der Gemische auf Temperaturen bis zu 120°C, insbesondere bei Temperaturen zwischen 5 und 50°C. Um eine gute Aushärtung der erfindungsgemässen Gemische bei niedrigen Temperaturen, beispielsweise zwischen 5 und 50°C zu erzielen, können die Gemische mit Härtungsbeschleunigern auf Basis von tertiären Aminen und/oder Phenolen und/oder Alkali- oder Erdalkalisalzen eingesetzt werden, wie beispielsweise 2,4,6-Tris-(dimethylaminomethyl)-phenol, Nonylphenol, Calcium- oder Magnesiumnitrat.

Ein weiterer Gegenstand der Erfindung sind auch die durch Härten der erfindungsgemässen Gemische erhaltenen Formstoffe oder Beschichtungen.

Wie eingangs erwähnt, zeichnen sich die aus den erfindungsgemässen Gemischen hergestellten Formstoffe und Beschichtungen durch eine hohe Chemikalienresistenz, insbesondere gegenüber Carbonsäuren, wie beispielsweise wässriger Essigsäure mit einem Gehalt an Essigsäure bis zu 30 Gew-%, aus.

### Herstellung der Polyaminoamide:

### Beispiel 1

5,83 g (0,03 Mol) Isophthalsäuredimethylester, 25,55 g (0,15 Mol) Isophorondiamin (IPD) und 13,45 g Benzylalkohol werden vermischt und allmählich erwärmt. Bei 55°C geht alles in Lösung. Die Temperatur wird weiter erhöht, wobei Methanol abdestilliert wird. Nach 2,25 Stunden (h) bei 150°C wird das Gemisch abgekühlt. Es werden 41,22 g (97 % der Theorie) eines Produktegemisches erhalten, das aus 30 Gew.-% Benzylalkohol und 70 Gew.% eines Polyaminoamidgemisches besteht, das neben dem Überschuss an IPD hauptsächlich die Bisamide der Formeln 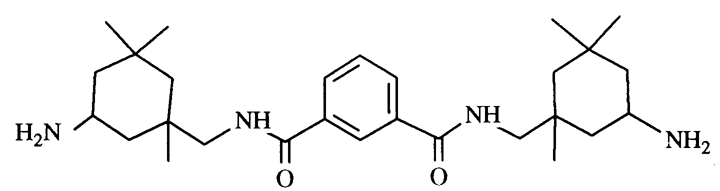 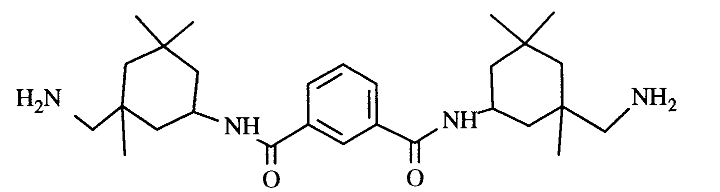
und deren Strukur- und Stereoisomeren enthält.

### Beispiel 2

6,5 g (0,033 Mol) Isophthalsäuredimethylester, 22,7 g (0,167 Mol) m-Xylylendiamin (MXDA) und 12,5 g Benzylalkohol werden vermischt und allmählich erwärmt. Bei 60°C geht alles in Lösung. Die Temperatur wird weiter erhöht, wobei Methanol abdestilliert wird. Nach 1 h bei 160°C wird das Gemisch abgekühlt. Es werden 38,08 g (96 % d. Th.) eines Produktegemisches erhalten, das aus 30 Gew.-% Benzylalkohol und 70 Gew.-% eines Aminoamidgemisches besteht, das neben dem Überschuss an MXDA hauptsächlich aus dem Bisamid neben wenig Monoamid der Formeln 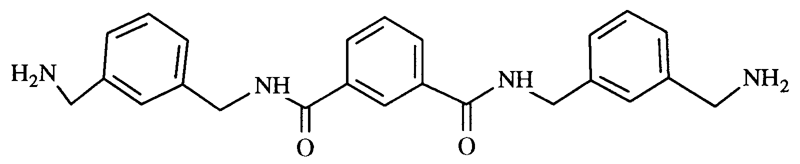 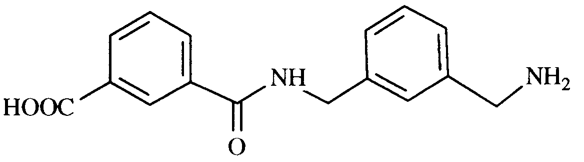
besteht.

### Beispiel 3

5 g (25,75 mMol) Isophthalsäuredimethylester, 41,4 g (129 mMol) Ancamine X2168 (MPCA) der Firma Anchor Chemical und 19,9 g Benzylalkohol werden während 1,5 h bei 150°C gerührt, wobei Methanol abdestilliert wird. Nach dem Abkühlen werden 64,36 g (100% d. Th.) eines Gemisches erhalten, das aus 30 Gew.-% Benzylalkohol und 70 Gew.-% eines Aminoamidgemisches besteht, das neben dem überschüssigen MPCA hauptsächlich aus den Bisamiden der Formeln 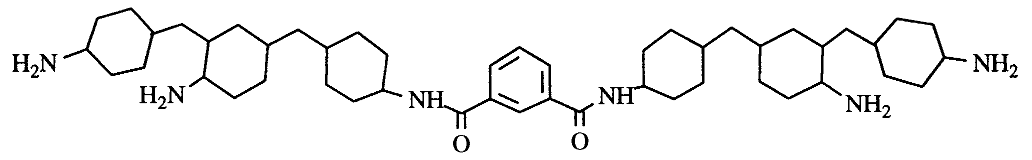 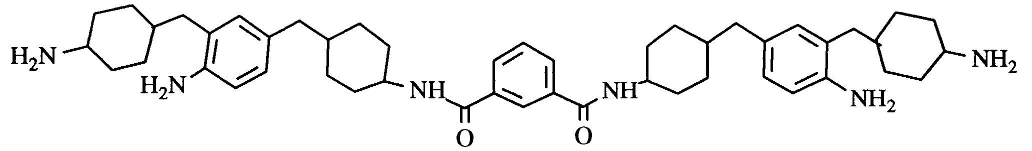
und deren Struktur- und Stereoisomeren besteht.

### Beispiel 4

5,83 g (0,03 Mol) Terephthalsäuredimethylester, 25,55 g (0,15 Mol) Isophorondiamin (IPD) und 13,45 g Benzylalkohol werden vermischt und allmählich erwärmt. Bei 90°C geht alles in Lösung. Die Temperatur wird weiter erhöht, wobei Methanol abdestilliert wird. Nach 110 Minuten (min) bei 152°C wird das Gemisch abgekühlt. Es werden 40,84 g (96 % d. Th.) eines Produktegemisches erhalten, das aus 30 Gew.-% Benzylalkohol und 70 Gew.-% eines Polyaminoamidgemisches besteht, das neben dem Überschuss an IPD hauptsächlich die folgenden Terephthalsäurebisamide neben wenig -monoamiden 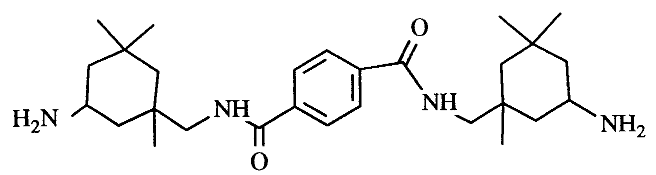 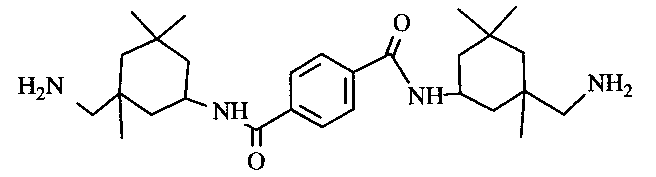 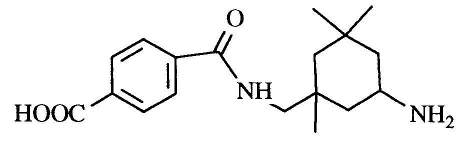
und 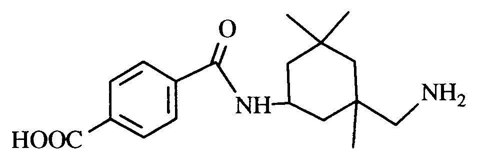
sowie deren Struktur- und Stereoisomeren enthält.

### Beispiel 5

5 g (25,75 mMol) Terephthalsäuredimethylester, 41,4 g (129 mMol) Ancamine X2168 (MPCA) und 19,9 g Benzylalkohol werden während 2,1 h bei 140°C gerührt, wobei Methanol abdestilliert wird. Nach dem Abkühlen werden 63,67 g (99% d. Th.) eines Produktegemisches erhalten, das aus 30 Gew.-% Benzylalkohol und 70 Gew.-% eines Aminoamidgemisches besteht, das neben dem überschüssigen MPCA hauptsächlich die Bisamide der Formeln 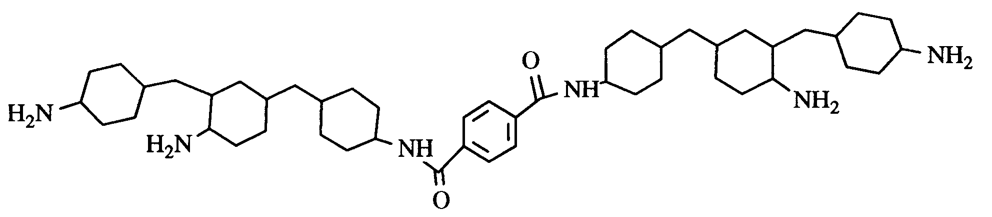 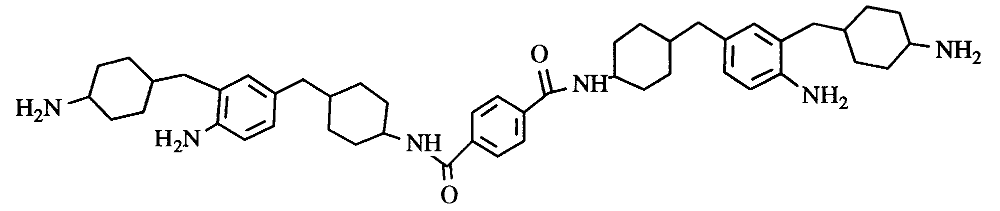
und deren Struktur- und Strereoisomeren enthält.

### Beispiel 6

9,71 g (0,05 Mol) Isophthalsäuredimethylester, 17,13 g (0,15 Mol) 1,2-Diaminocyclohexan (1,2-DACH, cis/trans-Gemisch) und 11,5 g Benzylalkohol werden vermischt und allmählich erwärmt. Bei 60°C geht alles in Lösung. Die Temperatur wird weiter erhöht, wobei Methanol abdestilliert wird. Nach 2 h 10 min bei 150°C wird das Gemisch abgekühlt. Es werden 33,34 g (95 % d. Th.) eines Produktegemisches erhalten, das aus 30 Gew.-% Benzylalkohol und 70 Gew.-% eines Aminoamidgemisches besteht, das hauptsächlich das Bisamid der Formel 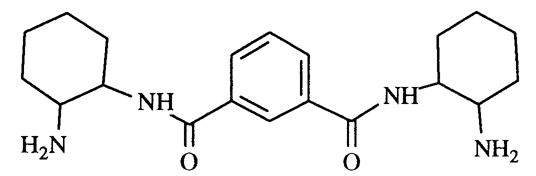
und dessen Stereoisomeren sowie einen Überschuss an 1,2-DACH enthält.

Das Isomerengemisch der Bisamide kann wie folgt rein hergestellt und isoliert werden: 6 g (30,9 mMol) Isophthalsäuredimethylester und 70,56 g (618 mMol) 1,2-DACH werden während 3 h bei 140°C gerührt, wobei Methanol abdestilliert wird. Nach dem Abkühlen werden 75,84 g eines flüssigen Rohproduktes erhalten. 10 g dieses Rohproduktes werden bei 100 - 110°C im Hochvakuum vom Überschuss an 1,2-DACH befreit. Der halbfeste Rückstand (1,74 g) wird mit Diethylether verrührt und die erhaltenen Kristalle werden abfiltriert. Der Niederschlag enthält gemäss ¹H-NMR-Spektrum ein Isomerengemisch aus dem Bisamid der oben angegebenen Formel.
¹H-NMR-Spektrum der Hauptkomponente (DMSO-d₆): 0,6 - 2,4 (16H, m, CH₂); 2,69 (2H, m, CH-NH₂); 3,56 (2H, m, CH-NH); 7,30 (1H, t); 7,86 - 8,00 (2H, m); 7,97 (1H, m); 8,40 (1H, m); 9,44 (2H, m, NH).
Massenspektrum (m/e): 97 (Basispeak 100%); 149 (21%); 200 (22%); 201 (14%); 244 (11%); 262 (16%); 322 (1%); 340 (6%); 358 (M+, 1%).

### Beispiel 7

9,0 g (46,3 mMol) Isophthalsäuredimethylester, 48,75 g (232 mMol) Bis-(4-aminocyclohexyl-)methan (PACM) und 24,75 g Benzylalkohol werden 3,2 h bei 160°C gerührt, wobei Methanol abdestilliert wird. Nach dem Abkühlen werden 79,7 g (100% d. Th.) eines Produktegemisches erhalten, das aus 30 Gew.-% Benzylalkohol und 70 Gew.-% eines Gemisches besteht, das neben dem Überschuss an PACM hauptsächlich das cis-/trans-Isomerengemisch des Bisamids der Formel 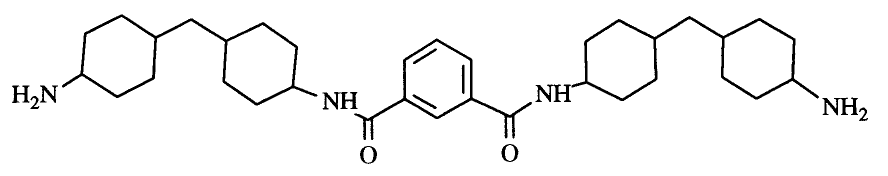
enthält.

### Beispiel 8

12 g (61,8 mMol) Terephthalsäuredimethylester, 44 g (309 mMol)
1,3-Bis-(aminomethyl-)cyclohexan (1,3-BAC) und 24 g Benzylalkohol werden wärend 3 h bei 160°C gerührt, wobei Methanol abdestilliert wird. Nach dem Abkühlen werden 75,2 g (99% d. Th.) eines farblosen, klaren Oels erhalten, das aus 30 Gew.-% Benzylalkohol und 70 Gew.-% eines Gemisches besteht, das neben dem überschüssigen 1,3-BAC hauptsächlich das Bisamid der Formel 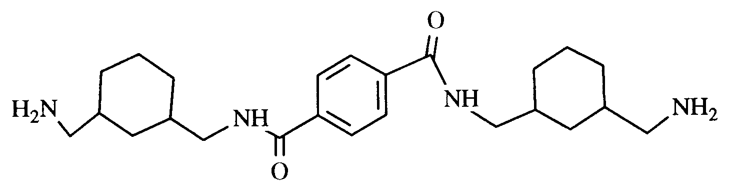
als Isomerengemisch enthält.

Dieses Produkt (Isomerengemisch cis/trans) kann wie folgt rein hergestellt und isoliert werden:
5 g (25,7 mMol) Terephthalsäuredimethylester und 73,25 g (515 mMol)
1,3-Bis-(aminomethyl-)cyclohexan (1,3-BAC) werden wärend 3 h. bei 140°C gerührt, wobei Methanol abdestilliert wird. Das Gemisch (77,55 g) wird 4 Wochen bei 5° gehalten, wobei ein Niederschlag entsteht. Die Suspension wird mit Methylenchlorid verrührt, abfiltriert und der Niederschlag mehrmals mit Methylenchlorid gewaschen und dann getrocknet.

### Beispiel 9

5 g (25,7 mMol) Terephthalsäuredimethylester und 70,13 g (515 mMol) leicht wasserhaltiges m-Xylylendiamin (MXDA) werden 3 h bei 140°C gerührt, wobei Methanol abdestilliert wird. Nach dem Abkühlen werden 74,12 g eines flüssigen Produktegemisches erhalten. 10 g dieses Gemisches werden durch Erwärmen im Hochvakuum vom überschüssigen MXDA befreit. Der Rückstand (1,14 g) wird mit heissem Ethanol verrührt. Der Niederschlag wird abfiltriert, mit Diethylether gewaschen und getrocknet. Das kristalline Produkt enthält gemäss ¹H-NMR-Spektrum 30 Gew.-% des Bisamids der Formel 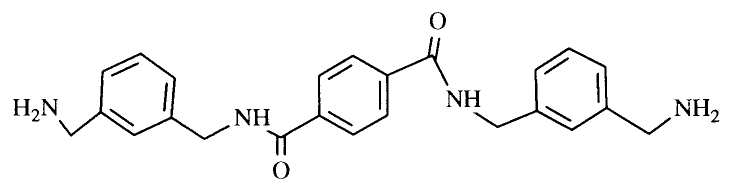
sowie 50 Gew.% des Monoamids der Formel 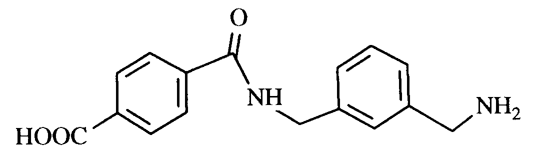
und etwa 20 Gew.-% Terephthalsäure.

### Beispiel 10

6,0 g (30,9 mMol) Terephthalsäuredimethylester und 70,56 g (618 mMol) 1,2-Diaminocyclohexan (1,2-DACH) werden während 3 h bei 140°C gerührt, wobei Methanol abdestilliert wird. Nach Abkühlen werden 75,71 g eines Produktegemisches erhalten, das bald einen Niederschlag bildet. Dieser wird abfiltriert, mit Pentan gewaschen und getrocknet. Umkristallisation aus heissem Toluol ergibt laut ¹H-NMR-Spektrum ein Isomerengemisch des Bisamids der Formel

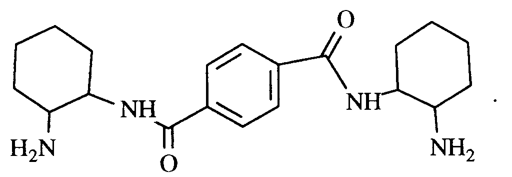

### Beispiel 11

12,0 g (60 mMol) Cyclohexan-1,4-dicarbonsäuredimethylester (cis-/trans-Gemisch), 40,81 g (300 mMol) m-Xylylendiamin (MXDA) und 22,63 g Benzylalkohol werden vermischt und allmählich erwärmt, wobei ab etwa 140°C Methanol abdestilliert wird. Nach 7 h bei 175°C wird das Gemisch abgekühlt. Es werden 68,4 g (96 % d.Th.) eines halbfesten Produktegemisches erhalten, das aus 30 Gew.-% Benzylalkohol und 70 Gew.-% eines Aminoamidgemisches besteht, das neben dem Überschuss an MXDA hauptsächlich aus dem cis-/trans-Isomerengemisch des Bisamids der Formel 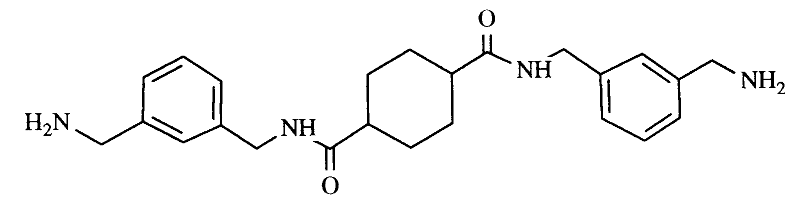
besteht.

### Beispiel 12

10,0 g (57,4 mMol) Adipinsäuredimethylester, 39,1 g (287 mMol) m-Xylylendiamin (MXDA) und 21,0 g Benzylalkohol werden vermischt und allmählich erwärmt, wobei ab etwa 140°C Methanol abdestilliert wird. Nach 7 h bei 180°C wird das Gemisch abgekühlt. Es werden 64,85 g (98 % d. Th.) eines halbfesten Produktegemisches erhalten, das aus 30 Gew.-% Benzylalkohol und 70 Gew.-% eines Aminoamidgemisches besteht, das neben dem Überschuss an MXDA hauptsächlich aus dem Bisamid der Formel 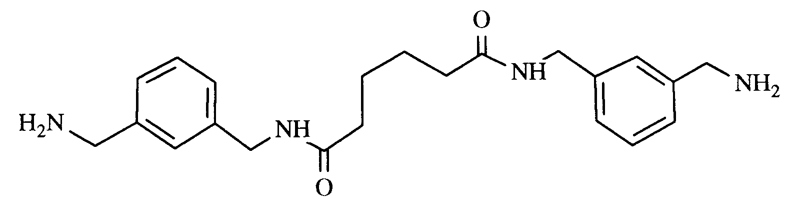
besteht.

### Beispiel 13

10,0 g (49,94 mMol) Cyclohexan-1,4-dicarbonsäuredimethylester (cis-/trans-Gemisch), 42,53 g (249,7 mMol) Isophorondiamin (IPD) und 22,51 g Benzylalkohol werden vermischt und allmählich erwärmt, wobei ab etwa 140°C Methanol abdestilliert wird. Nach 7,5 h Erhitzen bei 180°C wird das Gemisch abgekühlt. Es werden 70,96 g (99 % d.Th.) eines schwach gelblichen Produktegemisches erhalten, das aus 30 Gew.-% Benzylalkohol und 70 Gew.-% eines Aminoamidgemisches besteht, das neben dem Überschuss an IPD hauptsächlich aus den Bisamiden der Formeln 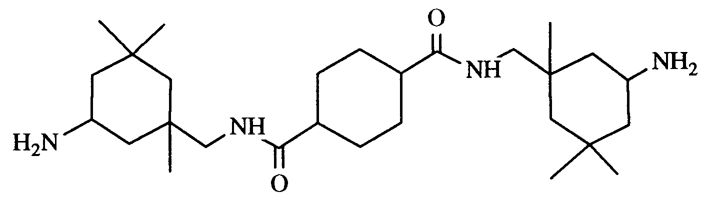 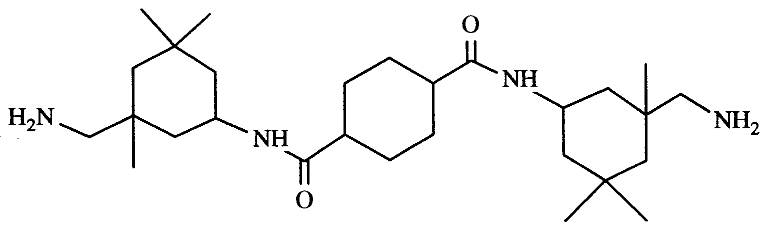
und deren Struktur- und Stereoisomeren besteht.

### Beispiel 14

10,0 g (57,4 mMol) Adipinsäuredimethylester, 48,93 g (287 mMol) Isophorondiamin (IPD) und 25,24 g Benzylalkohol werden vermischt und allmählich erwärmt, wobei ab etwa 140°C Methanol abdestilliert wird. Nach 7 h Erhitzen bei 180°C wird das Gemisch abgekühlt. Es werden 79,63 g (99 % d.Th.) eines schwach gelblichen Produktegemisches erhalten, das aus 30 Gew.-% Benzylalkohol und 70 Gew.-% eines Aminoamidgemisches besteht, das neben dem Überschuss an IPD hauptsächlich aus den Bisamiden der Formeln 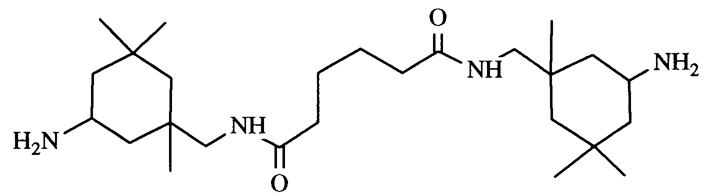 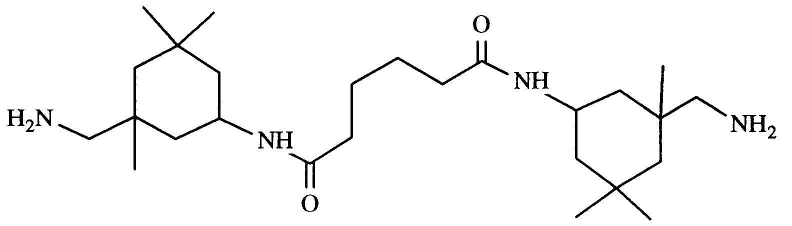
und deren Struktur- und Stereoisomeren besteht.

### Beispiel 15

202,5 g (1487 mMol) m-Xylylendiamin (MXDA) und 108,22 g Benzylalkohol werden vermischt und auf 50°C erwärmt. Dann werden 50,0 g (198,24 mMol)
Benzol-1,3,5-tricarbonsäuretrimethylester portionenweise zugegeben. Das Gemisch wird weiter erwärmt, wobei ab etwa 85°C vollständige Lösung eintritt. Das Gemisch wird während 7,5 h bei 180°C gerührt, wobei Methanol abdestilliert wird. Nach dem Abkühlen werden 342,35 g (100 % d.Th.) eines gelblichen Produktegemisches erhalten, das aus 30 Gew.-% Benzylalkohol und 70 Gew.-% eines Aminoamidgemisches besteht, das neben dem Überschuss an MXDA hauptsächlich aus dem Trisamid der Formel 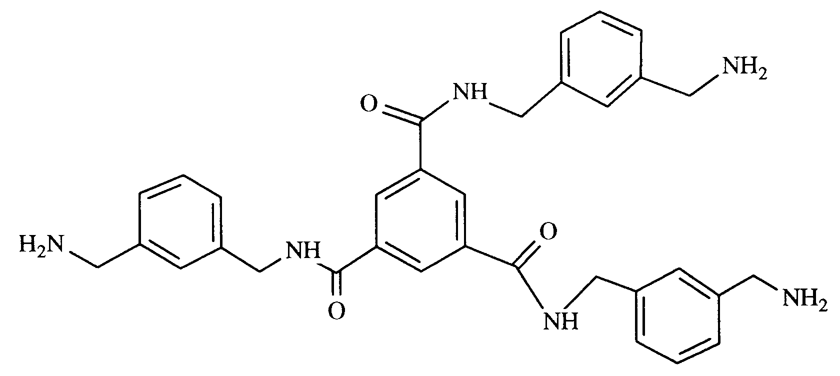
besteht.

### Beispiel 16

202,56 g (1189 mMol) Isophorondiamin (IPD) und 103,95 g Benzylalkohol werden vermischt und auf 50°C erwärmt. Dann werden 40,0 g (158,6 mMol)
Benzol-1,3,5-tricarbonsäuretrimethylester portionenweise zugegeben. Das Gemisch wird weiter erwärmt, wobei ab etwa 85°C vollständige Lösung eintritt. Das Gemisch wird während 7,5 h bei 180°C gerührt, wobei Methanol abdestilliert wird. Nach dem Abkühlen werden 330,09 g (100 % d.Th.) eines gelblichen halbfesten Produktegemisches erhalten, das aus 30 Gew.-% Benzylalkohol und 70 Gew.-% eines Aminoamidgemisches besteht, das neben dem Überschuss an MXDA hauptsächlich aus den Trisamiden der Formeln 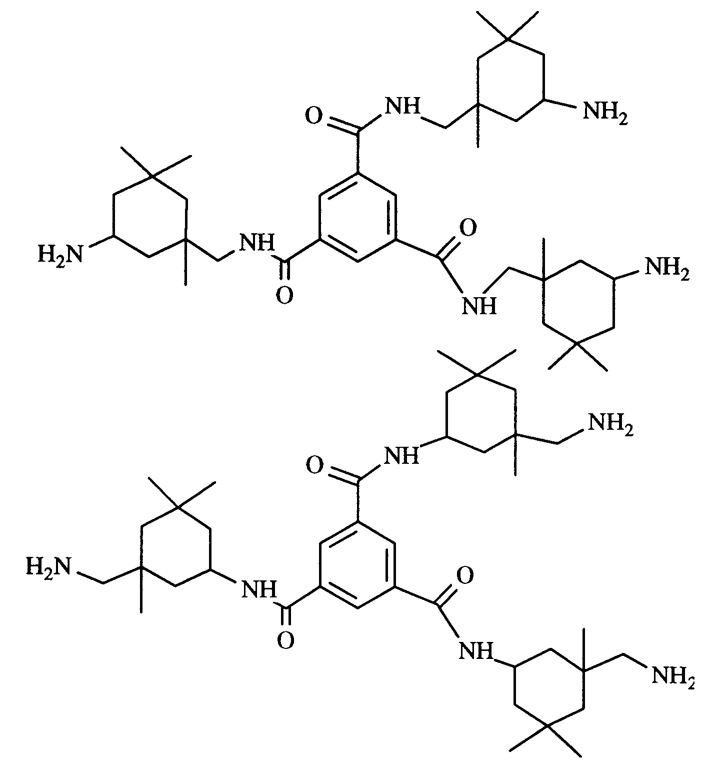
sowie deren statistisch verteilten Struktur- und Stereoisomeren besteht.

### Beispiel 17

12,0 g (43,43 mMol) Citronensäuretriethylester, 104,74 g (325,7 mMol) Ancamine X2168 (MPCA) und 50,03 g Benzylalkohol werden vermischt und allmählich erwärmt, wobei ab etwa 140°C Ethanol abdestilliert wird. Nach 2 h bei 180°C wird das Gemisch abgekühlt. Es werden 154,19 g (96 % d.Th.) eines Produktegemisches erhalten, das aus 30 Gew.-% Benzylalkohol und 70 Gew.-% eines Polyaminoamidgemisches besteht, das neben dem Überschuss an MPCA hauptsächlich aus den Trisamiden der Formeln 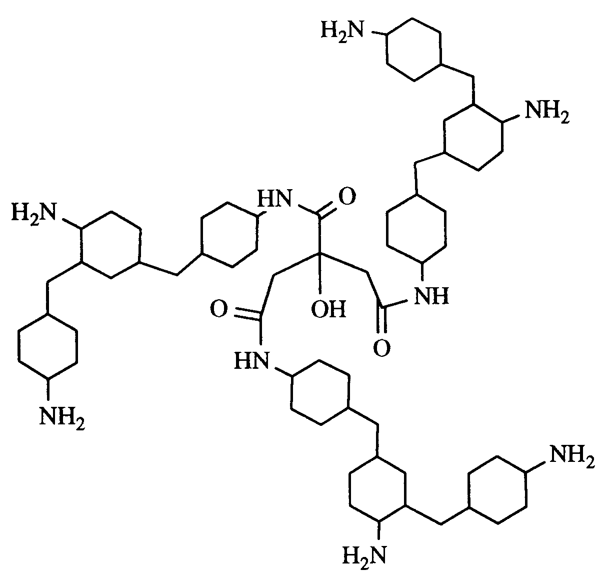 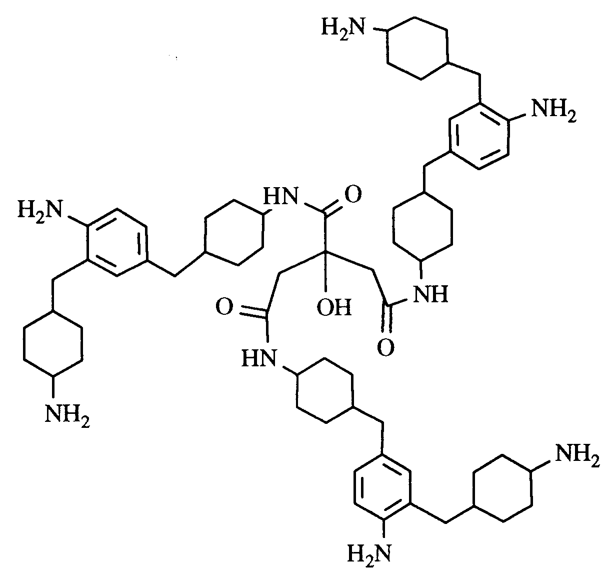
sowie deren statistisch verteilten Struktur- und Stereoisomeren besteht.

### Anwendungsbeispiele

### Beispiel I

2,5 g des Härters gemäss Beispiel 1 werden mit 0,125 g Salicylsäure während einer Stunde bei 60°C gerührt. Das klare Gemisch wird auf 20°C abgekühlt und mit 5,0 g Bisphenol A-diglycidylether (Epoxidgehalt = 5,25-5,40 Äquivalente/kg; Viskosität = 10000-12000 mPa·s) gründlich vermischt. Das klare Gemisch wird mittels Pinsel auf eine sandgestrahlte, entfettete Stahlplatte oder mittels Rakel (0,20 mm) auf eine Glasplatte aufgetragen. Nach wenigen Stunden bei 20°C entstehen klare, farblose und harte Schichten, die auf der Glasplatte nach einem Tag bei 20°C eine Persoz-Härte von 233 s aufweisen, gemessen mit einem Pendeldämpfungsprüfer TY 5853 (BYK-Chemie) nach Persoz. Nach 8 Tagen Durchhärtung bei 20°C ist die Persoz-Härte auf 315 s gestiegen. Die Schicht auf der Stahlplatte widersteht dann einer Belastung durch Benetzen mit 10%-iger wässriger Essigsäure während mindestens 2 Monaten, ohne Löcher zu bilden oder sich abzulösen.

Das oben hergestellte härtbare Gemisch härtet auch bei 100% relativer Luftfeuchtigkeit und einer Temperatur von 5°C oder von 20°C befriedigend aus, wobei keine oder nur eine sehr geringe Oberflächentrübung auftritt.

### Beispiel II

2,5 g des Härters gemäss Beispiel 2 werden erst mit 2,0 g Nonylphenol und dann mit 6,0 g Bisphenol A-diglycidylether (Epoxidgehalt = 5,25-5,40 Äquivalente/kg; Viskosität = 10000-12000 mPa·s) gründlich vermischt. Das klare Gemisch wird mittels Pinsel auf eine sandgestrahlte, entfettete Stahlplatte oder mittels Rakel (0,20 mm) auf eine Glasplatte aufgetragen. Nach wenigen Stunden bei 20°C entstehen klare, farblose und harte Schichten, die auf der Glasplatte nach einem Tag bei 20°C eine Persoz-Härte von 175 s ( mittels Pendeldämpfungsprüfer TY 5853) aufweisen. Nach 8 Tagen Durchhärtung bei 20°C ist die Persoz-Härte auf 226 s gestiegen. Die Schicht auf der Stahlplatte widersteht dann einer Belastung durch Benetzen mit 10%-iger wässriger Essigsäure während mindestens 3 Monaten, ohne Löcher zu bilden oder sich abzulösen.

Das oben hergestellte härtbare Gemisch härtet auch bei 100% relativer Luftfeuchtigkeit und einer Temperatur von 5°C oder von 20°C befriedigend aus, wobei keine oder nur eine sehr geringe Oberflächentrübung auftritt.

### Beispiel III

2,5 g einer 30%-igen Lösung des Härters gemäss Beispiel 3 werden mit 4,77 g Bisphenol A-diglycidylether (Epoxidgehalt = 5,25-5,40 Äquivalente/kg; Viskosität = 10000-12000 mPa·s) gründlich vermischt. Das klare Gemisch wird mittels Pinsel auf eine sandgestrahlte, entfettete Stahlplatte oder mittels Rakel (0,20 mm) auf eine Glasplatte aufgetragen. Nach wenigen Stunden bei 20°C entstehen klare, farblose und harte Schichten, die auf der Glasplatte nach einem Tag bei 20°C eine Persoz-Härte von 186 s aufweisen. Nach 8 Tagen Durchhärtung bei 20°C ist die Persoz-Härte auf 325 s (mittels Pendeldämpfungsprüfer TY 5853) gestiegen. Die Schicht auf der Stahlplatte widersteht dann einer Belastung durch Benetzen mit 10%-iger wässriger Essigsäure während mindestens 2 Monaten, ohne Löcher zu bilden oder sich abzulösen.

Das oben hergestellte härtbare Gemisch härtet auch bei 100% relativer Luftfeuchtigkeit und einer Temperatur von 5°C oder von 20°C befriedigend aus, wobei keine oder nur eine sehr geringe Oberflächentrübung auftritt.

### Beispiel IV

2,0 g des Härters gemäss Beispiel 1 und 0,5 g des Härters gemäss Beispiel 2 werden mit 6,0 g Bisphenol A-diglycidylether (Epoxidgehalt = 5,25-5,40 Äquivalente/kg; Viskosität = 10000-12000 mPa·s) gründlich vermischt. Das klare Gemisch wird mittels Pinsel auf eine sandgestrahlte, entfettete Stahlplatte oder mittels Rakel (0,20 mm) auf eine Glasplatte aufgetragen. Nach wenigen Stunden bei 20°C entstehen klare, farblose und harte Schichten, die auf der Glasplatte nach einem Tag bei 20°C eine Persoz-Härte von 211 s (mittels Pendeldämpfungsprüfer TY 5853) aufweisen. Nach 8 Tagen Durchhärtung bei 20°C ist die Persoz-Härte auf 295 s gestiegen. Die Schicht auf der Stahlplatte widersteht dann einer Belastung durch Benetzen mit 10%-iger wässriger Essigsäure während mindestens 2 Monaten, ohne Löcher zu bilden oder sich abzulösen.

Das oben hergestellte härtbare Gemisch härtet auch bei 100% relativer Luftfeuchtigkeit und einer Temperatur von 5°C oder von 20°C befriedigend aus, wobei keine oder nur eine sehr geringe Oberflächentrübung auftritt.

### Beispiel V

2,5 g des Härters gemäss Beispiel 18 werden erst mit 1,0 g 4-Nonylphenol und dann mit 4,79 g Bisphenol A-diglycidylether (Epoxidgehalt = 5,25 - 5,40 Äquivalente/kg; Viskosität = 10000 - 12000 mPa·s) gründlich vermischt. Das klare Gemisch wird mittels Pinsel auf eine sandgestrahlte, entfettete Stahlplatte oder mittels Rakel (0,20 mm) auf eine Glasplatte aufgetragen. Nach wenigen Stunden bei 20°C entstehen klare, farblose und harte Schichten, die auf der Glasplatte nach einem Tag bei 20°C eine Persoz-Härte von 95 s (gemessen mit einem Pendeldämpfungsprüfer TY 5853) aufweisen. Nach 8 Tagen Durchhärtung bei 20°C ist die Persoz-Härte auf 239 s gestiegen. Die Schicht auf der Stahlplatte widersteht dann einer Belastung durch Benetzen mit 10%-iger wässriger Essigsäure während mindestens 2 Monaten bei 20°C, ohne Löcher zu bilden oder sich abzulösen.

Das oben hergestellte härtbare Gemisch härtet auch bei 100 % relativer Luftfeuchtigkeit und einer Temperatur von 5°C oder 20°C befriedigend aus, wobei keine oder nur eine sehr geringe Oberflächentrübung auftritt.

## Patentansprüche

1. Polyaminopolyamide der Formel I 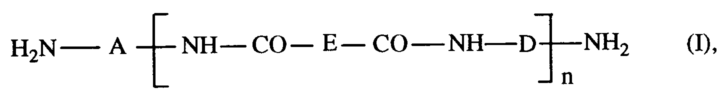 worin n eine Zahl von 1 bis 4 bedeutet,
A und D gleich oder verschieden sind und unabhängig voneinander je für einen Rest der Formeln 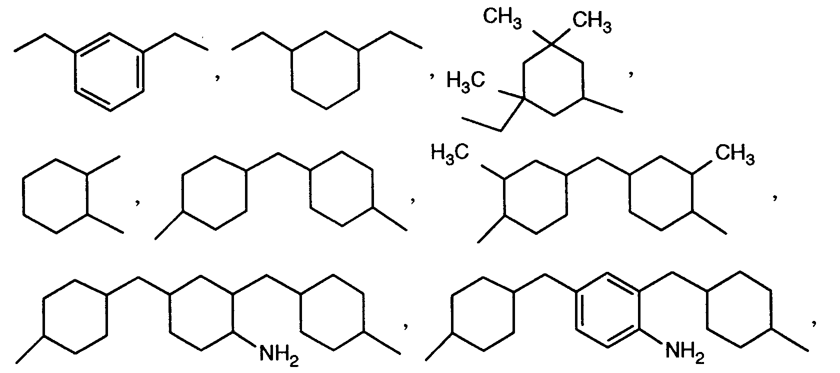 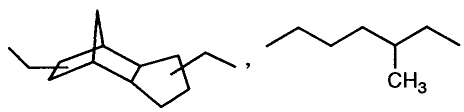 oder für ein Isomerengemisch aus den beiden Resten der Formel 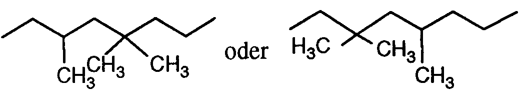 stehen und E einen Rest der Formel 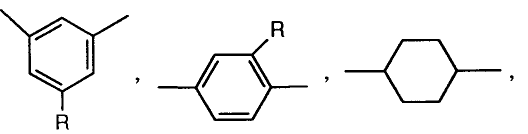 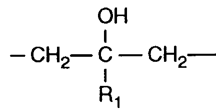 oder (̵CH₂ 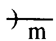 bedeutet, wobei R für ein Wasserstoffatom oder einen Rest der Formel -CO-NH-A-NH₂ oder -CO-NH-D-NH₂ steht, R₁ für einen Rest der Formel -CO-NH-A-NH₂ oder -CO-NH-D-NH₂ steht und m eine Zahl von 2 bis 12 bedeutet.

2. Polyaminopolyamide gemäss Anspruch 1, worin in Formel I
n eine Zahl von 1 bis 4 bedeutet,
A und D gleich oder verschieden sind und unabhängig voneinander je für einen Rest der Formeln 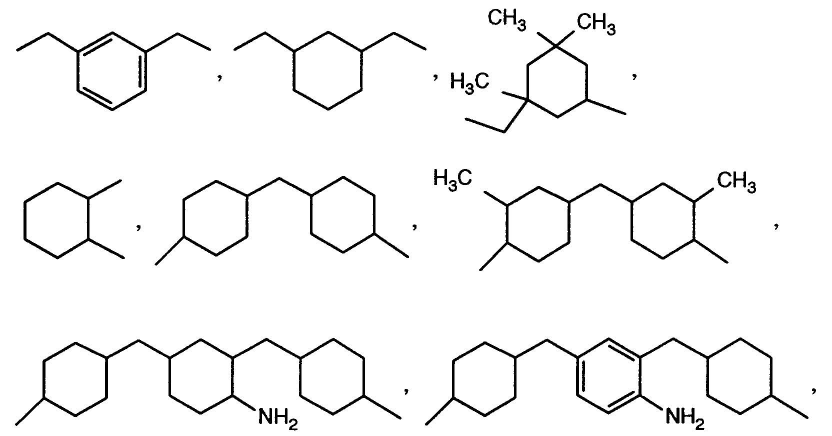 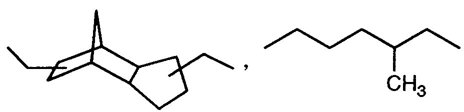 oder für ein Isomerengemisch aus den beiden Resten der Formel 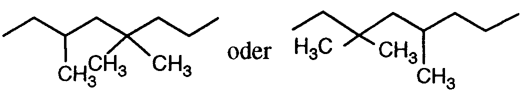 stehen und E einen Rest der Formel 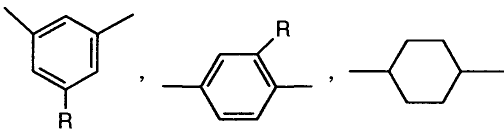 oder (̵CH₂ 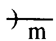 bedeutet, wobei R für ein Wasserstoffatom steht und m eine Zahl von 4 bis 12, vorzugsweise 6 bis 8, bedeutet.

3. Diaminopolyamide der Formel I gemäss Anspruch 1, dadurch gekennzeichnet, dass E einen Rest der Formeln 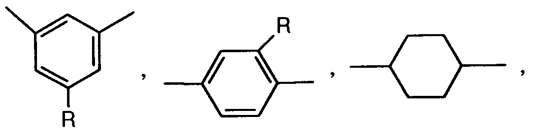 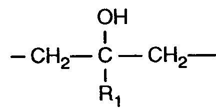 oder (̵CH₂ 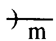 bedeutet, worin R für ein Wasserstoffatom steht, R₁ für einen Rest der Formel -CO-NH-A-NH₂ oder -CO-NH-D-NH₂ steht und m eine Zahl von 4 bis 12, vorzugsweise die Zahl 4, 6 oder 8, bedeutet.

4. Diaminopolyamide der Formel I gemäss Anspruch 1, dadurch gekennzeichnet, dass E einen Rest der Formeln 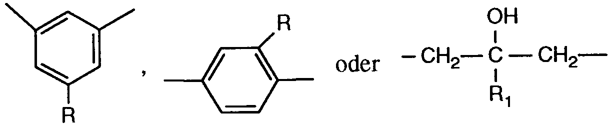 bedeutet, worin R für ein Wasserstoffatom steht und R₁ für einen Rest der Formel -CO-NH-A-NH₂ oder -CO-NH-D-NH₂ steht.

5. Polyaminopolyamide der Formel I gemäss Anspruch 1, dadurch gekennzeichnet, dass A und D unabhängig voneinander je einen Rest der Formeln 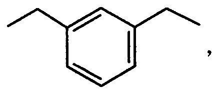 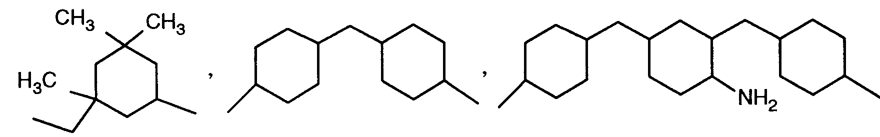 oder 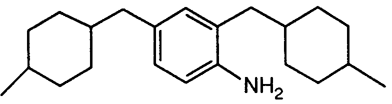 bedeuten.

6. Polyaminopolyamide der Formel I gemäss Anspruch 1, dadurch gekennzeichnet, dass A und D die gleiche Bedeutung haben.

7. Polyaminopolyamide der Formel I gemäss Anspruch 1, dadurch gekennzeichnet, dass n für die Zahl 1 steht.

8. Härtungsmittel für Epoxidharze, dadurch gekennzeichnet, dass es mindestens eine Verbindung der Formel I gemäss Anspruch 1 enthält.

9. Härtungsmittel für Epoxidharze gemäss Anspruch 8, das erhalten wird, indem man eine Di- oder Tricarbonsäure oder deren Ester der Formel II
R²OOC―E―COOR² (II),
worin E einen Rest der Formel 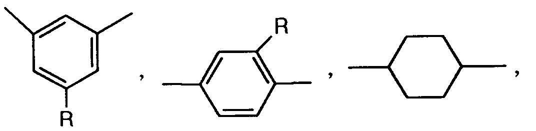 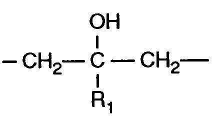 oder (̵CH₂ 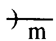 bedeutet, wobei R für ein Wasserstoffatom oder einen Rest der Formel -CO-NH-A-NH₂ oder -CO-NH-D-NH₂ steht, R₁ für einen Rest der Formel -CO-NH-A-NH₂ oder -CO-NH-D-NH₂ steht und m eine Zahl von 2 bis 12 bedeutet, mit einem Di- oder Triamin der Formel III und IV
H₂N―A―NH₂ (III) und H₂N―D―NH₂ (IV),
worin A und D die gleiche Bedeutung wie in Formel I gemäss Anspruch 1 haben, bei erhöhter Temperatur umsetzt, wobei man auf 1 Mol der Verbindung der Formel II 2 bis 20 Mole der Verbindungen der Formeln III und IV einsetzt.

10. Härtbares Gemisch, enthaltend
(a) ein Epoxidharz mit mehr als einer 1,2-Epoxidgruppe im Molekül und
(b) eine Verbindung der Formel I gemäss Anspruch 1.

11. Die durch Härten der Gemische gemäss Anspruch 10 erhaltenen Formstoffe oder Beschichtungen.
